(19) Europäisches Patentamt
European Patent Office
Office européen des brevets

(11) EP 0 749 951 A1

# (12) EUROPÄISCHE PATENTANMELDUNG

(43) Veröffentlichungstag:
**27.12.1996 Patentblatt 1996/52**

(51) Int. Cl.[6]: **C07C 51/48**, C07C 59/08, C07C 59/255, C07C 59/265

(21) Anmeldenummer: **96109505.6**

(22) Anmeldetag: **13.06.1996**

(84) Benannte Vertragsstaaten:
**AT CH DE DK ES FR GB IT LI**

(30) Priorität: **22.06.1995 DE 19522377**

(71) Anmelder: **BASF AKTIENGESELLSCHAFT**
**67056 Ludwigshafen (DE)**

(72) Erfinder:
- **Hansen, Hanspeter, Dr.**
  **67061 Ludwigshafen (DE)**
- **Vogel, Herbert, Prof. Dr.**
  **67069 Ludwigshafen (DE)**

## (54) Verfahren zur Gewinnung von Hydroxycarbonsäuren aus wässrigen Lösungen

(57) Die vorliegende Erfindung betrifft ein Verfahren zur Gewinnung von Hydroxycarbonsäuren durch Extraktion aus ihren verdünnten wäßrigen Lösungen und anschließende Destillation der so erhaltenen Extrakte, dadurch gekennzeichnet, daß man als Extraktionsmittel ein sekundäres Amid der allgemeinen Formel I

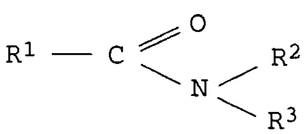

I

verwendet, in der $R^1$ Wasserstoff, Alkyl-, Cycloalkyl-, Aryl-, Aralkyl- oder Alkylhydroxygruppen und $R^2$ und $R^3$ unabhängig voneinander Alkyl-, Cycloalky-, Aryl-, Aralkyl- oder Alkylhydroxygruppen mit der Maßgabe bedeuten, daß die Summe der C-Atome von $R^1$, $R^2$ und $R^3$ 7 bis 14 beträgt.

EP 0 749 951 A1

Printed by Rank Xerox (UK) Business Services
2.13.10/3.4

## Beschreibung

Die vorliegende Erfindung betrifft ein neues Verfahren zur Gewinnung von Hydroxycarbonsäuren aus ihren wäßrigen Lösungen.

Neben den chemischen Synthesen werden Hydroxycarbonsäuren häufig auch durch enzymatische Verfahren hergestellt. Dabei fallen die Hydroxycarbonsäuren im Fermentationsmedium in Form von verdünnten wäßrigen Lösungen an. Um die meist ausgezeichnet wasserlöslichen Hydroxycarbonsäuren hieraus in reiner oder konzentrierter Form zu erhalten ist eine Reihe von aufwendigen Anreicherungsverfahren nötig.

Die Entfernung des Wassers durch direkte Destillation oder mit Hilfe eines wasserunlöslichen Schleppmittels ist wegen der hohen Energieeinsatzkosten meist nicht sinnvoll.

Aufgrund der guten Wasserlöslichkeit der Hydroxycarbonsäuren ist die Extraktion mit üblichen Lösungsmitteln wie Ethylacetat, Methylenchlorid oder Butanol nicht besonders effektiv, und befriedigende Ergebnisse lassen sich nur mit aufwendigen Mehrfachextraktionen erzielen.

Zur Lösung dieses Problems wurden Reaktivextraktionen vorgeschlagen, bei denen dem organischen Extraktionsmittel wasserunlösliche höhere zwei- oder dreiwertige Amine zugesetzt wurden, die mit den Hydroxycarbonsäuren die entsprechenden Ammoniumsalze bilden, die besser extrahiert werden können.

In Chem. Ing.-Techn. 58 (1986) Seite 308-317, wird beschrieben, daß sich der Verteilungskoeffizient von Salicylsäure im System Xylol/Wasser durch Zugabe eines sekundären Amins (LA-2) um den Faktor 50 vergrößern läßt.

In Chem. Ing.-Techn. 63 (1991) Seite 809-816 wird beispielsweise auf die Reaktivextraktion von Milchsäure mittels dem dreiwertigen Amin Alamin 336 (Tri-n-(C8-C10)-Amin) hingewiesen.

Die anschließende Aufarbeitung des Extraktes ist jedoch technisch schwierig, da die destillative Trennung von Hydroxycarbonsäuren und Aminen selbst bei reduziertem Druck noch relativ hohe Temperaturen erfordert, bei denen die Hydroxycarbonsäuren sich zersetzen oder unerwünschte Nebenreaktionen eingehen.

Im Falle von enantiomerenreinen Hydroxycarbonsäuren erfolgt unter diesen Bedingungen oft eine teilweise Racemisierung des Produktes, die der Anwendung dieses Verfahrens Grenzen setzt.

Aus DE 25 45 658 ist bekannt, daß sich die Carbonsäuren Ameisensäure, Essigsäure, Propionsäure uni Acrylsäure mit Amiden gut aus ihren wäßrigen Lösungen extrahieren lassen. Jedoch läßt sich in diesem Dokument kein Hinweis darauf finden, ob dieses Verfahren auch für andere als die vier oben angegebenen Carbonsäuren brauchbar ist.

Es bestand daher die Aufgabe, ein Verfahren zur Gewinnung von Hydroxycarbonsäuren aus ihren verdunnten wäßrigen Lösungen bereitzustellen, das die oben beschriebenen Nachteile nicht besitzt.

Es wurde gefunden, daß man Hydroxycarbonsäuren durch Extraktion aus ihren verdünnten wäßrigen Lösungen und anschließende Destillation der so erhaltenen Extrakte gewinnen kann, indem man als Extraktionsmittel ein sekundäres Amid der allgemeinen Formel I

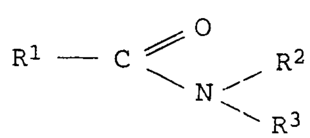

I

verwendet, in der $R^1$ Wasserstoff, Alkyl-, Cycloalkyl-, Aryl-, Aralkyl- oder Alkylhydroxygruppen und $R^2$ und $R^3$ unabhängig voneinander Alkyl-, Cycloalky-, Aryl-, Aralkyl- oder Alkylhydroxygruppen mit der Maßgabe bedeuten, daß die Summe der C-Atome von $R^1$, $R^2$ und $R^3$ 7 bis 14 beträgt.

Unter Hydroxycarbonsäuren sind solche organischen Säuren zu verstehen, die neben einer oder mehrerer COOH-Gruppen mindestens eine Hydroxygruppe enthalten. Das erfindungsgemäße Verfahren läßt sich beispielsweise auf Mono-, Di-, Tri- und Polyhydroxycarbonsäuren anwenden. Besonders geeignet ist das Verfahren für natürlich vorkommende Hydroxycarbonsäuren wie Glykolsäure, Milchsäure, Äpfelsäure, Weinsäure, Zitronensäure, Gluconsäure, Mandelsäure, Serin, Threonin und Tyrosin.

Ganz besonders gut eignet sich das Verfahren zur Gewinnung von Milchsäure, insbesondere auch von enantiomerenreiner Milchsäure.

Hinsichtlich der Reste R2 und R3 der Amide I sind solche Verbindungen oder deren Gemische besonders geeignet, die sich vom N-Ethyl-N-cyclohexylamin, N,N-Dicyclohexylamin, N,N-Dibutylamin, N-Methyl-N-benzylamin, N-Methylanilin, N-Ethylanilin, N,N-Diamylamin, N-Methyl-N-Ethylhexylamin, N-n-Butyl-N-cyclohexylamin, N-Methyl-N-2-heptylamin oder N-Propyl-N-cyclohexylamin ableiten.

Hinsichtlich des Rests R1 sind Formyl- und niedere Alkyl- bzw. Hydroxyalkylgruppen bevorzugt.

Da es manchmal zu einer Umamidierung des Amids I mit der Hydroxycarbonsäure kommen kann, kann es vorteilhaft sein als Rest R1 des Amids den gleichen Rest wie die Hydroxycarbonsäure zu wählen, damit eine stattfindende Umamidierung keinen störenden Einfluß auf das Produkt hat.

Bevorzugt werden solche Amide I verwendet, bei denen die Gesamtzahl der C-Atome der Reste R1, R2 und R3

kleiner oder gleich 10 ist.

Die Amide der Formel I sind entweder bekannt oder nach bekannten Methoden leicht zugänglich. In der Regel werden die Amide I ohne weitere Lösungsmittel eingesetzt, da so der Wirkungsgrad der Extraktion am höchsten ist. Soll jedoch eine Extraktion bei relativ niedriger Temperatur, d.h. unterhalb des Erstarrungspunkts des Amids I durchgeführt werden, so empfiehlt sich der Zusatz eines organischen Lösungsmittels, das eine Herabsetzung des Erstarrungspunktes bewirkt.

Ein Maß für die Eignung der Extraktionsmittel ist der Verteilungskoeffizient, der die Konzentration der Hydroxycarbonsäure im Extraktionsmittel im Verhältnis zur Konzentration der Hydroxycarbonsäure in Wasser angibt.

Je kleiner dieser Wert ist, umso größerer apparativer Aufwand ist für die Extraktion nötig.

Der Verteilungskoeffizient für Wasser spielt bei dem erfindungsgemäßen Verfahren keine so entscheidende Rolle, da das im Extrakt gelöste Wasser leicht zusammen mit dem Extraktionsmittel destillativ von der Hydroxycarbonsäure abgetrennt werden kann.

Die erforderliche Menge des Extraktionsmittels hängt von verschiedenen Parametern ab, darunter von der Temperatur, der Menge und Konzentration der Hydroxycarbonsäure, der Anzahl der Trennstufen sowie weiteren apparativen Gegebenheiten und ist dem Fachmann geläufig oder leicht für ihn bestimmbar.

Für die Extraktion kommt vorzugsweise ein Temperaturbereich von 0°C bis 100°C in Betracht. Bei den unteren Werten dieses Bereiches ist das Aufnahmevermögen des Extraktionsmittels für die Hydroxycarbonsäure zwar größer als bei höheren Temperaturen; dafür ist die Geschwindigkeit der Phasenscheidung langsamer. Das wirtschaftliche Optimum liegt in der Regel bei 40°C bis 80°C, bzw. es kann leicht durch eine Reihe von Routineversuchen ermittelt werden.

Die Tatsache, daß die Verteilungskoeffizienten bei höherer Temperatur deutlich niedriger sind als bei niedrigen Temperaturen kann zusätzlich ausgenützt werden, indem man eine Temperaturwechselextraktion durchführt.

Hierbei extrahiert man die wäßrige Hydroxycarbonsäurelösung mittels Amid I bei tiefer Temperatur, z.B. 20°C bis 40°C, und unterwirft die Extraktphase bei hoher Temperatur, z.B. 60°C bis 80°C, einer Rückextraktion mit Wasser.

Die Extraktion läßt sich kontinuierlich oder diskontinuierlich durchführen. In der technischen Anwendung wird die kontinuierliche Extraktion im Gegenstromverfahren bevorzugt.

Gewünschtenfalls läßt sich durch Verwendung mehrstufiger Extraktionsapparate, beispielsweise Batterien von Mischern und Scheidern oder Füllkörperkolonnen, die dem Fachmann prinzipiell geläufig sind, die Wirksamkeit noch steigern.

Das wesentliche Merkmal der vorliegenden Erfindung liegt nicht in der an sich bekannten Extraktionstechnik, sondern in der Art des Extraktionsmittels.

Nach dem Extraktionsschritt wird die extrahierte Phase in einer dem Fachmann geläufigen Art und Weise destillativ in Hydroxycarbonsäure und Extraktionsmittel sowie gegebenenfalls Wasser getrennt. Als Ergebnis des Gesamtverfahrens erhält man die Hydroxycarbonsäure in reiner Form oder wahlweise als konzentrierte Lösung.

Das erfindungsgemäße Verfahren läßt sich sowohl auf die Gewinnung chemisch oder biotechnologisch hergestellten Hydroxycarbonsäure anwenden, die in Form von wäßrigen Lösungen vorliegen. Es ist weiterhin auch für die Abwasseraufreinigung, wenn es auf die Entfernung von Hydroxycarbonsäure ankommt, geeignet. Besonders geeignet ist es zur Gewinnung von Hydroxycarbonsäure aus Fermentationsbrühen.

Das vorliegende Verfahren ermöglicht eine beträchtliche Einsparung an Energie und Investitionskosten sowohl im Vergleich zu anderen Extraktionsverfahren als auch im Vergleich zur destillativen Auftrennung. Die nachfolgenden Beispiele dienen der weiteren Veranschaulichung der Erfindung.

Beispiel 1

Da die Eignung der Extraktionsmittel im wesentlichen von Verteilungskoeffizienten

$$K = \frac{\text{Konzentration der Säure in der organischen Phase}}{\text{Konzentration der Säure in der wäßrigen Phase}}$$

abhängt, wurde dieser Koeffizient wie folgt ermittelt:

50 g einer wäßrigen 7 %igen Lösung der entsprechenden Hydroxycarbonsäure wurden mit 50 g Extraktionsmittel bei 40°C bis zur Gleichgewichtseinstellung gerührt. Danach wurde der Rührer abgestellt und so lange gewartet, bis die organische und die wäßrige Phase sich klar getrennt hatten.

Aus der Säurekonzentration, die durch Titration ermittelt wurde, wurde der Verteilungskoeffizient gebildet.

Die folgende Tabelle gibt eine Übersicht über die Verteilungskoeffizienten einiger erfindungsgemäßer und im Vergleich dazu herkömmlichen Extraktionsmittel an:

| Extraktionsmittel | Säure | K bei 40°C |
|---|---|---|
| N,N-Dibutylformamid | Milchsäure | 1,37 |
| " | Äpfelsäure | 1,36 |
| " | Citronensäure | 2,07 |
| N,N-Dibutylacetamid | Milchsäure | 1,52 |
| N,N-Dipropylpropionsäureamid | Milchsäure | 1,23 |
| N,N-Dibutylpropionsäureamid | Milchsäure | 1,00 |
| N,N-Di-n-butylmilchsäureamid | Milchsäure | 0,65 |
| **Herkömmliche Extraktionsmittel** | | |
| Benzol | Milchsäure | < 0,01 |
| Ethylacetat | Milchsäure | 0,29 |
| Chloroform | Milchsäure | 0,01 |
| i-Butanol | Milchsäure | 0,84 |
| i-Butanol | Citronensäure | 0,52 |

Beispiel 2

Dieses Beispiel zeigt die Temperaturabhängigkeit des K-Wertes von Milchsäure im System N,N-Di-n-butylformamid / Wasser.

Die Durchführung zur Bestimmung des K-Wertes erfolgte wie in Beispiel 1 angegeben.

| Temperatur in °C | K-Wert |
|---|---|
| 40 | 1,37 |
| 60 | 1,24 |
| 80 | 1,15 |

Beispiel 3

Dieses Beispiel zeigt die Konzentrationsabhängigkeit des K-Wertes von Citronensäure bei 40°C im System N,N-Di-n-butylformamid / Wasser.

Die Durchführung zur Bestimmung des K-Wertes erfolgte wie in Beispiel 1 angegeben.

| Konzentration der Citronensäure im Gleichgewicht im Wasser bei 40°C in Massenprozent | K-Wert |
|---|---|
| 0,5 | 2,70 |
| 1,1 | 2,47 |
| 2,5 | 2,07 |

## Patentansprüche

1. Verfahren zur Gewinnung von Hydroxycarbonsäuren durch Extraktion aus ihren verdünnten wäßrigen Lösungen und anschließende Destillation der so erhaltenen Extrakte, dadurch gekennzeichnet, daß man als Extraktionsmittel ein sekundäres Amid der allgemeinen Formel I

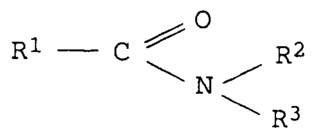

I

verwendet, in der $R^1$ Wasserstoff, Alkyl-, Cycloalkyl-, Aryl-, Aralkyl- oder Alkylhydroxygruppen und $R^2$ und $R^3$ unabhängig voneinander Alkyl-, Cycloalky-, Aryl-, Aralkyl- oder Alkylhydroxygruppen mit der Maßgabe bedeuten, daß die Summe der C-Atome von $R^1$, $R^2$ und $R^3$ 7 bis 14 beträgt.

2. Verfahren nach Anspruch 1, dadurch gekennzeichnet, daß eine Monohydroxymonocarbonsäure extrahiert wird.

3. Verfahren nach Anspruch 2, dadurch gekennzeichnet, daß Milchsäure extrahiert wird.

4. Verfahren nach Anspruch 1, dadurch gekennzeichnet, daß als Extraktionsmittel Di-n-butylformamid verwendet wird.

5. Verfahren nach Anspruch 1, dadurch gekennzeichnet, daß die verdünnte wäßrige Lösung eine Fermentationsbrühe ist.

Europäisches Patentamt

# EUROPÄISCHER RECHERCHENBERICHT

Nummer der Anmeldung
EP 96 10 9505

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | US-A-4 251 671 (ALTER ET AL.)<br>* Spalte 1, Zeile 40 - Zeile 45 *<br>* Anspruch 1 *<br>--- | 1,5 | C07C51/48<br>C07C59/08<br>C07C59/255<br>C07C59/265 |
| A | US-A-2 578 698 (HANFORD)<br>* Spalte 3, Zeile 17 - Zeile 30 *<br>* Anspruch 1 *<br>----- | 1 | |

| RECHERCHIERTE SACHGEBIETE (Int.Cl.6) |
|---|
| C07C |

Der vorliegende Recherchenbericht wurde für alle Patentansprüche erstellt

| Recherchenort | Abschlußdatum der Recherche | Prüfer |
|---|---|---|
| DEN HAAG | 26.September 1996 | Klag, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)